# EUROPEAN PATENT SPECIFICATION

(11) **EP 2 104 763 B1**
(45) Date of publication and mention of the grant of the patent: **29.03.2017**
(21) Application number: 07851962.6
(22) Date of filing: 27.12.2007
(51) Int. Cl.: D06M 10/08, D06M 16/00, D06M 10/10, D06M 14/18, A01N 43/50, A01N 43/52, D06M 10/02, D06M 14/10, A01N 33/12, D06M 14/12, D06M 14/22, D06M 14/28, D06M 14/30, D06M 15/227, D06M 15/233, D06M 15/267, D06M 15/564, A61L 2/232

(54) **SUBSTRATE WITH ANTIMICROBIAL COATING**
SUBSTRAT MIT ANTIMIKROBIELLER BESCHICHTUNG
SUBSTRAT AVEC REVÊTEMENT ANTIMICROBIEN

(30) Priority: 29.12.2006 EP 06077344
(43) Date of publication of application: 30.09.2009
(73) Proprietor: Nederlandse Organisatie voor toegepast- natuurwetenschappelijk onderzoek TNO, 2595 DA 's-Gravenhage (NL)
(72) Inventor: ALKEMA, Duurt, Pieter, Willem, NL-2288 GJ Rijswijk (NL); SIMOR, Marcel, NL-2288 GJ Rijswijk (NL); NOORT, Daniel, NL-2288 GJ Rijswijk (NL); TROMP, Maarten, Cornelis, NL-2288 GJ Rijswijk (NL); VAN HOOFT, Petrus, Albertus, Venantia, NL-2288 GJ Rijswijk (NL); FIALA, Ales, NL-3069 LN Rotterdam (NL)
(74) Representative: V.O.
(86) International application number: PCT/NL2007/050702
(87) International publication number: WO 2008/082293

(56) References cited:
- EP-A2- 0 591 024
- WO-A-2005/110626
- WO-A1-2006/111711
- WO-A2-2005/123891
- CERNAKOVA L ET AL: "Low-cost plasma activation and chitosan immobilization onto polypropylene nonwovens" PROCEEDINGS OF 14TH ANNUAL INTERNATIONAL TANDEC NONWOVENS CONFERENCE, 11 November 2004 (2004-11-11), pages 2.6-1-2.6-8, XP008090107
- SHI Z ET AL: "Antibacterial activity of polymeric substrate with surface grafted viologen moieties" BIOMATERIALS, ELSEVIER SCIENCE PUBLISHERS BV., BARKING, GB, vol. 26, no. 5, February 2005 (2005-02), pages 501-508, XP004522670 ISSN: 0142-9612
- WAFA D M ET AL: "Atmospheric plasma-aided biocidal finishes for nonwoven polypropylene fabrics. II. functionality of synthesized fabrics" JOURNAL OF APPLIED POLYMER SCIENCE, vol. 103, no. 3, 13 November 2006 (2006-11-13), pages 1911-1917, XP002443379
- VIRK R K ET AL: "PLASMA AND ANTIMICROBIAL TREATMENT OF NONWOVEN FABRICS FOR SURGICAL GOWNS", TEXTILE RESEARCH JOURNAL, SAGE PUBLICATIONS, LONDON, GB, vol. 74, no. 12, 1 December 2004 (2004-12-01), pages 1073-1079, XP001217796, ISSN: 0040-5175
- SHI Z ET AL: "Antibacterial activity of polymeric substrate with surface grafted viologen moieties", BIOMATERIALS, ELSEVIER SCIENCE PUBLISHERS BV., BARKING, GB, vol. 26, no. 5, 1 February 2005 (2005-02-01), pages 501-508, XP004522670, ISSN: 0142-9612, DOI: 10.1016/J.BIOMATERIALS.2004.02.060

## Description

The invention is directed to a process for preparing a substrate with an antimicrobial coating, to the use of a plasma treatment in the preparation of an antimicrobial coating, to the use of specific antimicrobial compounds, to a substrate with an antimicrobial coating and to the use of such substrate.

For a wide range of applications it is desirable to provide substrates with antimicrobial properties. A typical way of providing antimicrobial properties to a substrate, such as textile, is by treating the substrate with an antimicrobial agent. In the art, the antimicrobial agent is typically applied from solution by wet processing techniques such as sol-gel, soaking, spraying, dipping, fluid-flow, padding, printing, spin coating, and dip coating. Application of antimicrobial coatings using wet processing techniques are for instance known from US-B-6 368 361, EP-A-0 908 553, WO-A-2004/050132, US-A-2003/0 056 297, US-A-2003/0 106 162, and US-A-2003/0 088 923. These wet processing techniques have the disadvantage that they are relatively environmentally unfriendly, since large amounts of chemicals and solvents are required and high energy costs are involved due to heating and drying steps. Furthermore, the physical properties of the substrate may be disadvantageously affected by the applied coating. In addition, some wet deposition processes are difficult to scale-up due to the complicated multi-step processing.

Černáková et al. (Proceedings 14th Annual International Tandec Nonwovens Conference, 11 November 2004. pages 2.6-1 to 2.6-8) discloses that chitosan immobilised at polypropylene nonwoven fabrics results in an inhibition of activities towards the growth of *Bacillus subtillis* and *Escherichia coli*. Shi et al. (Biomaterials 2005, 26(5), 501-508) describe a method for preparing poly(ethylene therephthalate) with an assymetric viologen antimicrobial active compound, which comprises pre-treatment of the poly(ethylene therephthalate) with low pressure argon plasma, exposure to air and subsequent UV-induced graft copolymerisation of the viologen. Wafa et al. (Journal of Applied Polymer Science 2006, 103(3), 1911-1917) describe polypropylene biocidal fabric with antibacterial agents synthesized by a plasma-aided graft copolymerization technique

Object of the present invention is to overcome at least one of the above-mentioned disadvantages and to enhance performance of antimicrobial coatings by plasma treatment. This object has been met by the process of the invention according to which an antimicrobial coating is prepared on the surface of a substrate using a plasma treatment. Accordingly, the invention is directed to a process for preparing a substrate with an antimicrobial coating comprising:
- providing a substrate;
- subjecting a surface of the substrate to a plasma environment, wherein said plasma environment is applied by surface dielectric barrier discharges; and
- depositing an antimicrobial active compound having the ability to destroy at least some types of micro-organisms on the surface of the substrate.

The inventors found that the physical properties of the substrate, such as weight, strength, feel, and breathability are hardly affected by the process of the invention. The plasma treatment is more environmentally friendly than the traditional wet processing. Furthermore, the process of the invention can be advantageously carried out at atmospheric pressure.

Surprisingly the plasma treatment does not necessarily cause the antimicrobial active compound to lose its activity. In addition, it was found that the antimicrobial coatings obtained by the plasma treatment of the invention had unexpected improved properties over antimicrobial coatings obtained by conventional wet processing techniques.

The use of a plasma environment for depositing a polymer layer on a substrate material is for example known from EP-A-1 090 178, US-B-6 551 950 and WO-A-03/084681 that describe a method of coating a surface with oil and/or water repellent polymer layers. Suitably, low power pulsed plasma polymerisation is used in order to produce well-adhered coatings which exhibit excellent water and oil repellency but not antimicrobial functionality.

The use of a plasma environment for depositing a polymer layer on a substrate material is also known from the non-prepublished international patent application WO-A-2007/008063. However, this document is directed to nanoparticle containing polymer coatings and does not disclose a reactive antimicrobial active compound.

A way to achieve antimicrobial and multifunctional textiles is disclosed in WO-A-02/079563 and US-A-2003/0_056_297. The multifunctional textile composition comprises a textile having an antimicrobial functionality and a chemical agent attached thereto to impart an additional functionality, which includes waterproof finishing, soil repellent finishing, fire resistance finishing, wrinkle free finishing, anti-UV finishing, and antistatic finishing. Main limitation of the inventions is that the antimicrobial functionality is a property of the textile composition, which limits the variety of substrate materials. The multifunctional textile of the present invention may be prepared by deposition of a coating on virtually any substrate material.

The term "antimicrobial" as used in this application is meant to refer to the ability to destroy at least some types of micro-organisms.

The term "plasma" as used in this application is meant to refer to a partially ionised gas that represents a chemically active environment, which consists of active species such as electrons, ions, radicals, metastables and photons.

The term "plasma polymerisation" as used in this application is meant to refer to the procedure, in which polymerisable materials, stimulated through a plasma, condense as polymers.

The term "surface" as used in this application is meant to refer to outer surfaces of a substrate, but also to outer surfaces of fibres in a substrate, inner surfaces of porous fibres in a substrate, and inner surfaces of pores in a substrate.

The term "textile" as used in this application is meant to refer to a thin, flexible material made of any combination of cloth, fibre, or polymer.

The term "cloth" as used in this application is meant to refer to a thin, flexible material made from yarns.

The term "yarn" as used in this application is meant to refer to a continuous strand of fibres.

The term "fibre" as used in this application is meant to refer to a unit of matter, either natural, such as cotton, synthetic, such as polyester, or a combination thereof, which forms the basic element of, for example, fabrics, and textile structures. A fibre itself may have a porous structure with voids.

In accordance with the invention an antimicrobial coating is applied to a substrate. Thereafter an antimicrobial active compound is deposited (preferably reacted) on the surface of the substrate, before, while or after said surface is subjected to a plasma treatment.

In accordance with the invention, the plasma sources are surface dielectric barrier discharges (DBDs). In such surface DBD, the electrode structure of the plasma source comprises two electrodes arranged on opposite sides of a dielectric. Coplanar surface DBD is a special case of surface DBD in which metallic parts of electrodes are embedded in a dielectric and are not in direct contact with plasma, thus resulting in a longer lifetime of the electrodes.

Surface DBD plasma sources can generate a stable atmospheric pressure plasma not only in helium but, in nearly any gas mixture. Surface DBD plasma sources are very suitable for the treatment of surfaces and for the treatment of textile materials in particular. The reason for this is that in surface DBD plasma sources the plasma channels are parallel with a substrate surface and plasma is thus in a good contact with the surface. A further advantage of surface DBD plasma sources is that all surfaces, not only outer surfaces but also inner surfaces, are treated by plasma.

As an example, in the case of a fibrous substrate, such as various textiles, material is deposited around individual fibres that are on the surface but also in the bulk of the substrate. The consequence is that an enormous surface area may be covered. Further, voids between fibres are not filled and the fabric keeps its breathability.

A plasma jet and microwave plasma sources are particularly suitable for treating three-dimensional and/or non-flat substrates.

In one embodiment, the plasma treatment comprises a so-called plasma assisted grafting technique. This is a two-step process comprising plasma activation of the surface of the substrate followed by the exposure of the surface to a liquid or gaseous precursor, which may also be a gasified liquid or an atomised (sublimated) solid. Plasma activation of the substrate surface comprises hydrogen abstraction, radical formation and introduction of new functional groups from the plasma environment. New functional groups may also be introduced on the substrate surface from the surrounding air after plasma activation. The plasma activation results in a reactive activated surface.

In the second step of the plasma assisted grafting technique, a antimicrobial active compound is deposited on the surface after the surface is activated by the plasma. The antimicrobial active compound can undergo a conventional free radical reaction onto the activated surface of the substrate. The result is a very thin antimicrobial coating on the surface of the substrate.

The antimicrobial active compound may be provided on the surface in liquid or gaseous form. It is also possible to provide the antimicrobial active compound by a conventional wet processing technique, *i.e.* from solution. The term "antimicrobial active compound" is meant to include a composition of two or more compounds which can react to form a compound having antimicrobial activity. Such a composition of two or more compounds can react for instance before being deposited onto the substrate surface, during being deposited onto the substrate surface or after being deposited onto the substrate surface.

It was surprisingly found that antimicrobial coatings prepared by the plasma assisted grafting technique have a better antimicrobial activity than antimicrobial coatings prepared by conventional wet processing. For example antimicrobial and hydrophilic coatings were deposited by dipping preceded by a plasma pre-treatment. Plasma activation in nitrogen plasma with and without water vapour enhanced the antimicrobial performance of the coating by more than one order of magnitude.

According to another embodiment, which may optionally be combined with the first embodiment, the antimicrobial active compound is provided on the surface of the substrate in the plasma environment. The reactive antimicrobial active compound is then plasma polymerised on the surface of the substrate, resulting in a very thin antimicrobial coating on the surface of the substrate, for example with a thickness from several nanometers to hundreds of nanometres.

Polymers formed by the process of plasma polymerisation can have different chemical and physical properties from those formed by conventional polymerisation. Plasma polymerised films can be highly cross-linked and can, therefore, have many appealing characteristics such as thermal stability, chemical inertness, mechanical toughness and negligible ageing. Also the washing-off characteristics can be enhanced.

In contrast to the conventional wet processing techniques, the plasma polymerisation process of this embodiment of the invention advantageously does not require liquid baths comprising toxic or harmful chemicals. Further, no heating, drying and/or curing activities are needed thereby reducing operational costs.

The plasma polymerisation process has also been identified as plasma assisted vapour deposition, plasma enhanced vapour deposition, plasma (assisted) chemical vapour deposition and plasma enhanced chemical vapour deposition.

In the third embodiment, which may optionally be combined with previous embodiments, the plasma treatment comprises a so-called plasma induced polymerisation technique. This is a two-step process in which an antimicrobial active compound is provided on the surface of substrate followed by the exposure of the said surface to plasma environment.

The plasma induced polymerisation has also been identified as plasma induced grafting. An advantage of this embodiment is that it is not necessary that the antimicrobial active compound comprises a reactive group. The plasma environment or the activated substrate surface can comprise highly energetic radicals that are able to react even with non-reactive substances.

Plasma conditions can be found such that antimicrobial coatings prepared by plasma polymerisation and plasma induced polymerisation have a better antimicrobial activity than antimicrobial coatings prepared by conventional wet processing. A plasma activation of the substrate prior to plasma polymerising or plasma induced polymerising the antimicrobial active compound further enhances the antimicrobial performance.

It is advantageous that the plasma treatment is carried out under substantially atmospheric pressure. Cost for providing low pressure circumstances, such as for example required in the case of metal sputtering or metal evaporation techniques, can thus be avoided.

The resulting antimicrobial coating on the surface of the substrate is very thin, preferably less then 500 nm, more preferably 50-200 nm, most preferably 10-100 nm. Such thin coatings impart the desired properties to the substrate without significantly affecting the original characteristics of the original substrate, such as for instance breathability, feel and softness.

A major advantage of the present invention is that further functionalities can be introduced on the surface of the substrate in a minimum of processing steps. This may be achieved for instance by using multifunctional compounds. It is also possible to use different functional chemicals simultaneously with the reactive antimicrobial active compound or separately before or after deposition of the reactive antimicrobial compound in the process according to the invention in order to include additional functionalities.

Additional substrate functionalities that may be introduced or influenced are for instance hydrophobicity, dirt and soil repellence, oleophobicity, hydrophilicity, anti-static, anti-UV and flame retardation and resistance properties.

Nanoparticle additives, such as zinc oxide (ZnO), titanium dioxide (TiO₂), or silver (Ag) nanoparticles may also be included in the polymer coating. These additives can provide for example UV-protection or self-cleaning functionality to the substrate or further improve the antimicrobial functionality of the substrate. The nanoparticle additives may be any structure with a size in the nanometer and micrometer range. Those structures include for example nanoparticles, nanofibres and/or nanotubes or a combination of them, such as nanotubes with attached and/or embedded nanoparticles and/or nanoparticles with one or more elements, such as a drug, dye and/or fragrance encapsulated. Further the nanoparticle additives can comprise either one type of nanoparticle additive or a mixture of nanoparticle additives. Preferably, the nanoparticle additive comprises a metal and/or a metal oxide in order to result in specific enhanced features of the substrate. Instead of using metals and/or metal oxides, other organic, inorganic nanoparticle additives or inorganic-organic composites could be used. Nanoparticles are preferably deposited, if allowed by the structure of the substrate, on the level of individual fibres and on inner surfaces of pores of a substrate thus covering an enormous surface area. Nanoparticles can be deposited for example by plasma polymerisation, plasma assisted grafting or plasma induced grafting (polymerization).

The above-mentioned embodiments of the invention may be further combined with conventional wet processing techniques.

Irrespective of the way by which the antimicrobial coatings are applied according to the present invention, they are strongly bound to the surface of the substrate and maintain their antimicrobial activity.

The substrate can be any substantially flat material, such as non-woven or woven textile, a nanofibre non-woven, felt, yarn, fibre, paper, foil, membranes, leather, polymers, wood and/or ceramics. Preferably, the substrate comprises yarns and fibres. Particularly preferred substrates are textiles, yarns and fibres.

Preferably, the antimicrobial active compound comprises a reactive group. Said reactive group can be a linker attached to a known or new antimicrobial compound or can be part of a known or new antimicrobial compound.

The antimicrobial active compound may comprise a quaternary ammonium moiety. Preferably the antimicrobial active compound comprises an optionally substituted aromatic heterocycle with at least one nitrogen atom. Examples of such aromatic heterocycles are five or six membered rings with at least one nitrogen atom, such as imidazoles, triazoles, tetrazoles, pyridines, pyrroles, pyrimidines, pyridazine, pyrazine, purine, or (benzo-fused) derivatives thereof. The aromatic heterocycle may be substituted with one or more functional groups, such as a hydroxyl or an amino group. A preferred aromatic heterocycle is an imidazole or derivative thereof.

The reactive group is preferably a (meth)acrylate or derivative thereof, a hydroxyl or an amino group.

The reactive group may be bound through an isocyanate, glycidyl, an alcohol, an epoxide, a vinyl or an allyl moiety. It is also possible that the reactive group is linked by a spacer. The spacer may be a flexible carbon chain of typically 1-12 carbon atoms, preferably 2-8 carbon atoms.

Preferably, the antimicrobial active compound is a quaternary ammonium compound according to general formula (I) 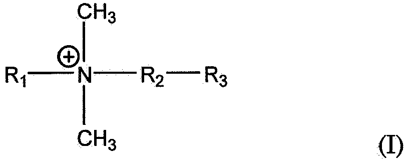
wherein R₁ is a C₃₋₁₂ alkyl group, preferably a C₅₋₉ alkyl group, more preferably a C₇ or C₈ alkyl group,
wherein R₂ is an optionally substituted C₁₋₆ chain, preferably a C₂₋₄ chain, and
wherein R₃ is chosen from the group consisting of an acrylate, a diisocyanate, a diol, an ethylene, and a styrene.
The counter ion can be a halogen ion, preferably chloride or bromide.

Another preferred antimicrobial active compound is a quaternary imidazole compound according to general formula (II) 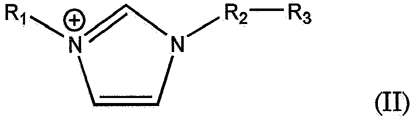 wherein R₁, R₂ and R₃ have the above identified meanings.

In case R₃ is an acrylate, the quaternary ammonium compounds and the imidazole compound may be provided on the surface of the substrate together with acrylic acid or (meth)acrylate monomers, such as butylmethacrylate, to provide a copolymer. The molar ratio between the antimicrobial agent and the acrylate monomer is preferably from 1:10 to 1:2, more preferably from 1:9 to 1:3.

It was found that the above-mentioned quaternary ammonium and quaternary imidazole compounds show a significant better antimicrobial activity than the commercially available antimicrobial agent DOW 5700 (octadecyldimethyl(3-trimethoxysilylpropyl)ammoniumchloride), also known as Aegis™, which has the following structure. 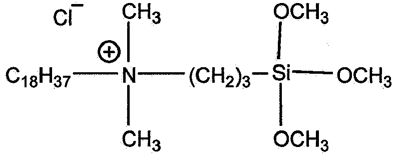 The above-mentioned quaternary ammonium and quaternary imidazole compounds also have a significant better antimicrobial activity than the commercially available silver-based antimicrobial agent Silpure, and compounds according to general formula (I) and (II) wherein the R₁ alkyl group has more than twelve carbon atoms.

It was further found that antimicrobial active compounds comprising a spacer located at R₂ based on poly(propylene oxide) or a polyethylene oxide or polytetramethyleneoxide showed enhanced antimicrobial behaviour, probably due to better adsorption of proteins to the surface.

Some examples of preferred antimicrobial compounds are shown in Table I.

**Table 1 Examples of preferred quaternary ammonium compounds.**

| | |
|---|---|
| 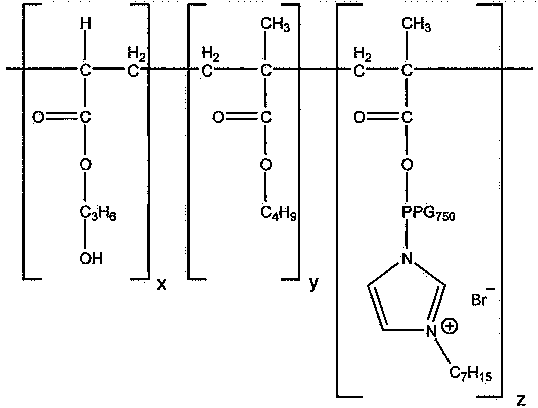 | x=2, y=1 and z=1 |
| 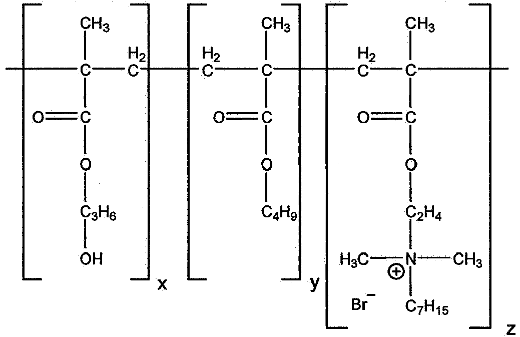 | x=1, y=2 and z=1 |
| 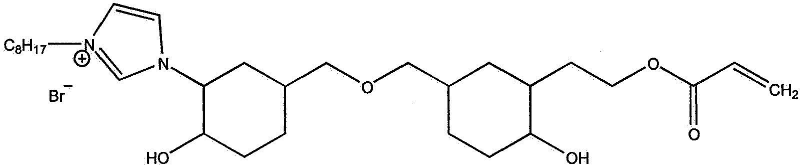 | |
| 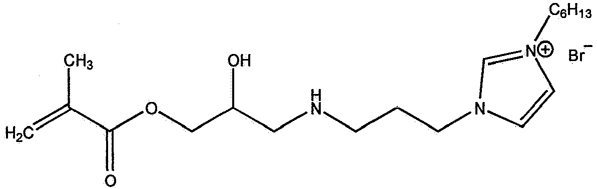 | |
| 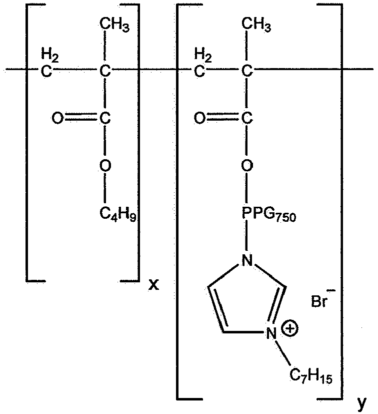 | x=2 and y=1 |
| | PPG750 = polypropylene glycol with a MW of 750 |
| 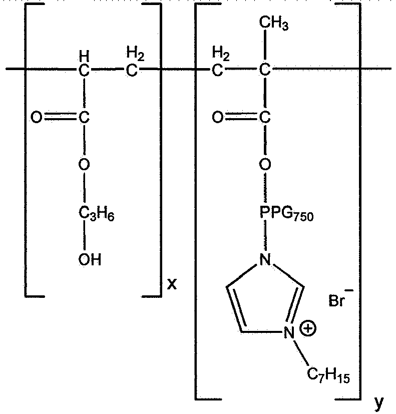 | x=1 and y=1 |
| | or |
| | x=4 and y=1 |
| | PPG750 = polypropylene glycol with a MW of 750 |
| 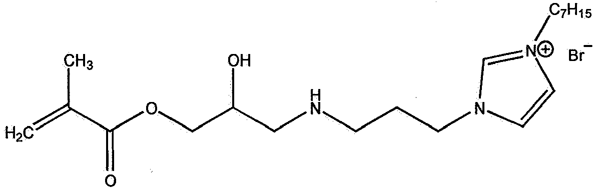 | |
| 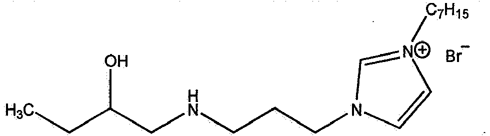 | |
| 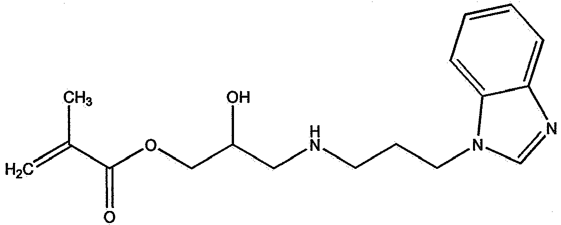 | |
| 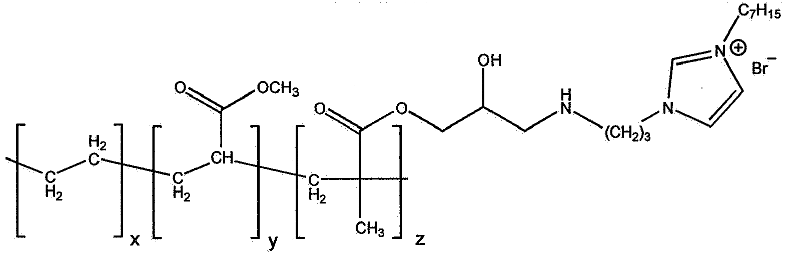 | x=5-15% |
| | y=70-90% |
| | z=5-15% |

Alternatively, the chemical compound to be deposited on the substrate is composed of at least two groups, wherein at least a first group is activated by the plasma species e.g. radicals such as N, O, O²⁻, HO₂, OH, which improves and/or provides binding of the chemical compound to the surface of the material to be treated, and/or said activated group forms a polymer; and wherein at least a second group has antimicrobial activity, but shows essentially no or only a minimal activation by plasma, or, alternatively, of which the antimicrobial activity is introduced or enhanced by plasma. The advantage of using such chemical compounds is that the antimicrobial activity is not destroyed by plasma and/or the subsequent binding and/or polymerisation.

The antimicrobial active compound is advantageously combined with an agent that provides another functionality to the coating in order to obtain a multifunctional coating. Suitable agents include for instance colour pigments for colouration to withstand fading, UV agents to withstand fading and degradation in products exposed to significant UV light, agents to make the surface of the substrate hydrophilic, hydrophobic or oleophobic; agents to make the product flame retardant or flame resistant, agents to improve printability, agents to modify frictional properties, agents to promote adhesion, bioactive agents and/or agents to make the product anti-static.

The antimicrobial active compound may be combined with the other functional agent in a single compound, but it is also possible that the other functional agent is a separate compound that is applied together with the antimicrobial active compound on the surface of the substrate.

In a further aspect, the invention is directed to the use of a plasma treatment in the preparation of an antimicrobial coating having the ability to destroy at least some types of micro-organisms for enhancing the antimicrobial properties of said coating, wherein said plasma treatment comprises a plasma assisted grafting technique, and wherein said plasma treatment is carried out under atmospheric pressure.

The invention is further directed to a substrate obtainable by the process of the invention, wherein the antimicrobial active compound is selected from the compounds listed in Table 1. As mentioned hereinabove, it was found that substrates with a polymer coating obtained by the assistance of plasma treatment in accordance with the invention not only have improved properties over a substrate with a polymer coating obtained by conventional wet processing techniques, but are also different in general structure, in the sense that much thinner coating layers are obtained.

A substrate obtainable by the process of the invention can be advantageously used to enhance the functionality of existing products, or they can be further processed into or directly used as for instance products against biological and/or chemical hazards and for personal protection products for example for police and military personnel, a packaging material, filters, face masks, anti-allergic products, cleaning products, hygiene articles, sports and apparel textiles, footwear, leather and technical textiles. Technical textiles according to a definition published by the textile institute, is defined as textile material and products manufactured primarily for their technical performance and functional properties rather than their aesthetic or decorative characteristics.

| **Table- 2** | | | | |
|---|---|---|---|---|
| **Construction Textile** | **Medical Textile** | **Industrial Articles** | **Mobile Textile** | **Protective Textile** |
| • Geotextile for under water and under ground construction | • Hygiene articles and Cosmetics | • Composite fabrics as alternative material | • Automobile interior design | • Defense textile |
| | • Hospital liners | | • Transportation tarpaulin | • Technical goods |
| • Textile for civil Engineering (reinforcing, roof and tents) | • Surgical articles and dressing | • Ape article and dressing | • Aviation and space travel | • Heat, cold fire, chemical protection |
| | | • Filter fabrics and sieves | | |
| | | | • Tyre, V-belt and drive belts | • Weather protection and sport clothing |
| • Agro covering | | • Polishing textiles (sanding, cleaning cloths) | • Transport belt, conveyer belts | |
| • Screening and Sound insulation | | | • Seal, brake lining | |
| | | • Roller covering Packing material Dyestuff carrier circuit board | | |

Technical textile is the most intelligent use of the textile material technically used by the industries of non-conventional character in high tech and high performance application starting from auto motive engineering to building and personal protection. As can be seen in the above Table 2, the vast application includes advertising, agriculture, automobile, aviation, civil engineering, chemical, electrical industries, leather, medical, environmental, protection, etc.

The substrate obtainable by the process of the invention may thus also be used for example for interior design of automobile, ship, aviation and space travel, carpets, leather and textiles for business and home use such as for example seat covers, curtains towels and linen, textiles for medical purposes such as for example bandages, medical garment, bed linen and filling material for example for furniture and screens. Historical textile, leather and paper can be treated by the process of invention to achieve antimicrobial protection together with for example protection against UV radiation.

In yet a further aspect, the invention is directed to the use of a substrate of the invention in building and/or construction, or as filling material, or for protection against biological and/or chemical hazards and/or for personal protection products, for example for police and military personnel.

### Example

### EXAMPLE 1 - General method for determining antibacterial activity

As the antibacterial activity depends on the nature of samples that are coated, treated samples and a clean (not treated) sample was always tested together. *Escherichia coli* ATCC 11775 was used for testing. At T=0 hours the total living bacteria, which was possible to clean from the material, were counted. After 24 hours of contact time at 37°C the surviving bacteria were counted again. With the numbers of bacteria at T=0 hours and T=24 hours, the antibacterial activity was calculated

### Method

### Pieces of cloth

Treated samples and one clean sample of cloth were used. The samples of cloth were divided into four equal pieces and every piece was placed in a sterile Petridish.

### Culture

*Escherichia coli* ATCC 11775 was cultured overnight at 37°C and 150 rpm in Brain Heart Infusion broth (BHI). 1 ml of overnight culture was added to 10 ml of BHI. This pre-culture was incubated for 2 hours at 37°C and 150 rpm.

### Contamination piece of cloth

All four pieces of cloth were contaminated with a 100 µl pre-culture. The drops were evenly spread over each piece of cloth with a spreader. Three pieces of cloth were incubated for 24 hours at 37°C while drying-up was prevented. One piece of cloth was prepared immediately (i.e. at T=0 hours). After the incubation, the surviving rate was determined. The pieces of cloth were placed in 5 ml physiological salt (FZ) or BHI. The tubes were vortexed for 2 minutes. From the FZ, a 10 fold dilution series was made in FZ.

### Plating

From each dilution 100µl was spread on a Tryptic Soy Agar plate (TSA) or Luria broth agar plate (LB). The plates were incubated for 18 hours at 37°C. After 18 hours the already formed colonies were counted. The plates were returned to the incubator for another 3 days at 37°C. After 3 days the colonies were counted again.

### Desired quantified results

The number of (surviving) bacteria was expressed as colony forming units (cfu). The antibacterial activity (R-value) was calculated as: log cfu T0 (control) - log cfu T24 (test material).

### EXAMPLE 2-Antabacterial activity of a coating of the invention

Plasma assisted grafting of poly(ethylene-co-methylacrylate-co-glycidylmethacrylate modified with N-hexylated 1-(3-aminopropyl)-imidazole was used for the deposition of antimicrobial and hydrophilic coating on a cotton substrate.

Dipping of cotton into the chemical and subsequent drying yielded a destruction efficiency of *Escherichia coli* (R of 4.1). Dipping of cotton into the same chemical, but performed after plasma activation in nitrogen plasma and nitrogen-water vapour plasma resulted in an R value of 4.6 and 5.2, respectively. Plasma activation was done with the help of surface dielectric barrier discharge.

### EXAMPLE 3

Plasma assisted grafting of N-(2-hydroxy-3-methacryloyl-propyl)-N'-heptyl-imidazolebromide was used to for the deposition of antimicrobial and hydrophilic coating on a polyester substrate. Treatment was performed like described in EXAMPLE 2.

Dipping of treated substrates into the chemical after the plasma activation in nitrogen (for 10 and 60 s) resulted in R values larger than 5.5 for polyester, as it can be seen in Table 3.

**Table 3**

| Chemical applied | Treat. time [s] | Bacteria concentration | | | antimicrobial activity R |
|---|---|---|---|---|---|
| | | initial | after 24 hours | | |
| | | | Specimen A | Specimen B | |
| no | - | 3.3.10⁶ | 9.2.10⁷ | 9.0.10⁷ | - |
| yes | - | 3.3.10⁶ | 2.4.10⁷ | 1.8.10⁷ | - |
| | 10 | 3.3.10⁶ | <10 | <10 | >5.5 |
| | 60 | 3.3.10⁶ | <10 | <10 | >5.5 |

## Claims

1. Process for preparing a substrate with an antimicrobial coating comprising:
- providing a substrate;
- subjecting a surface of the substrate to a plasma environment, wherein said plasma environment is applied by surface dielectric barrier discharges; and
- depositing an antimicrobial active compound having the ability to destroy at least some types of micro-organisms on the surface of the substrate.

2. Process according to claim 1, wherein at least part of the antimicrobial active compound is deposited on the surface of the substrate:
- simultaneously with subjecting the surface of the substrate to the plasma environment;
- after the surface of the substrate has been subjected to the plasma environment; or
- before the surface of the substrate having the applied antimicrobial active compound is subjected to the plasma environment.

3. Process according to any of claims 2, wherein the antimicrobial active compound is polymerised.

4. Process according to any one of the preceding claims, wherein the antimicrobial active compound comprises a quaternary ammonium moiety.

5. Process according to any one of the preceding claims, wherein the antimicrobial active compound comprises an optionally substituted aromatic heterocycle with at least one nitrogen atom, preferably selected from the group consisting of imidazoles, triazoles, tetrazoles, pyridines, pyrroles, pyrimidines, pyridazine, pyrazine, purine or a derivative thereof.

6. Process according to any one of the preceding claims, wherein the antimicrobial active compound is selected from: 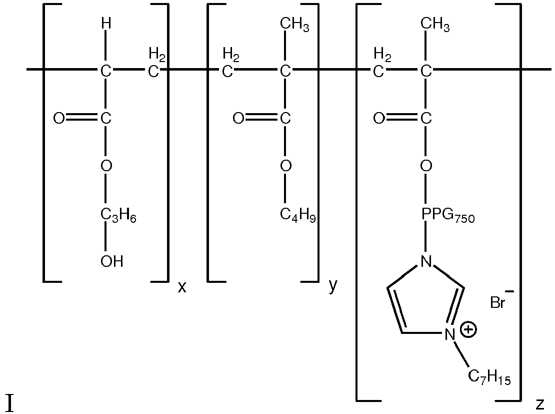 x = 2, y =1 and z = 1; 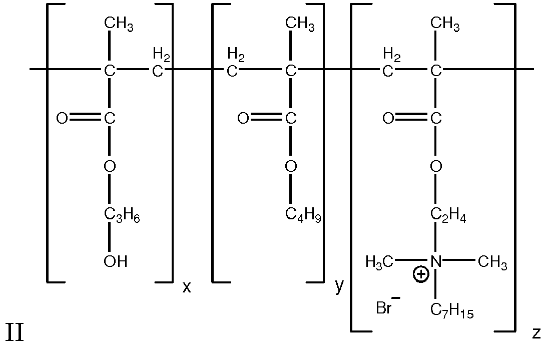 x = 1, y = 2 and z = 1; 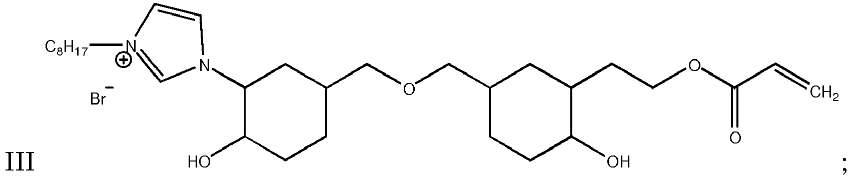 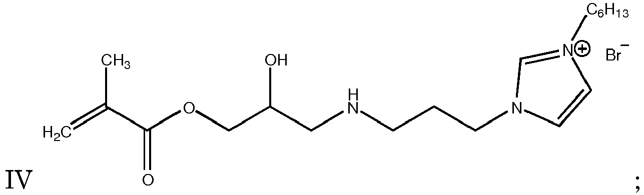 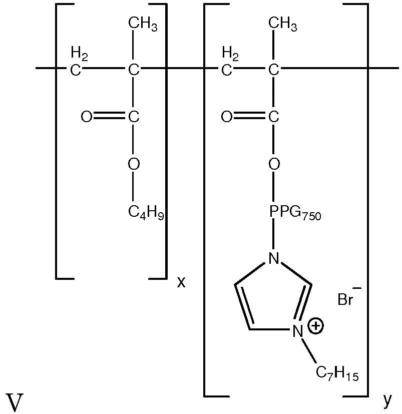 x = 2 and y = 1, PPG₇₅₀ = polypropylene glycol with a MW of 750; 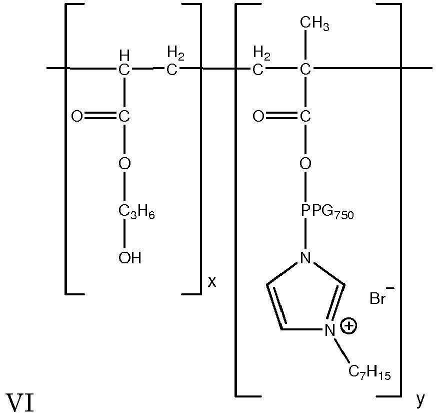 x = 1 or 4 and y = 1, PPG₇₅₀ = polypropylene glycol with a MW of 750; 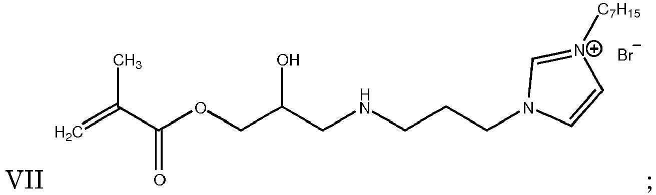 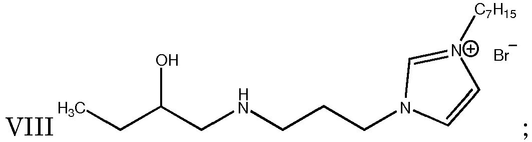 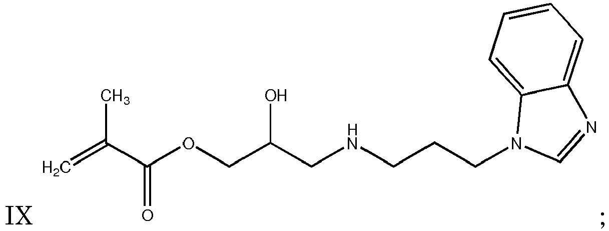 and 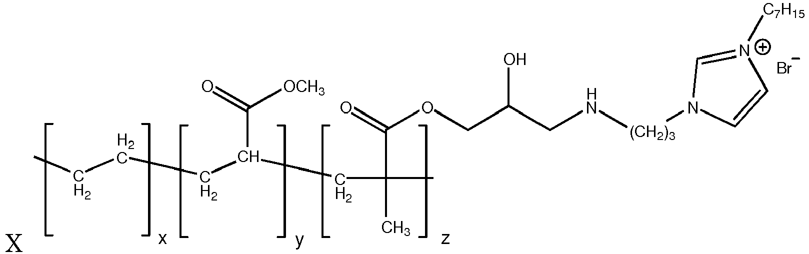 x = 5-15 %, y = 70-90 %, z = 5-15 %.

7. Process according to any one of the preceding claims, wherein the antimicrobial active compound comprises a reactive group selected from the group consisting of a (meth)acrylate or a derivative thereof, a hydroxyl or an amino group, preferably the reactive group is bound through an isocyanate, glycidyl, an alcohol, an epoxide, a vinyl or an allyl moiety.

8. Process according to any one of the preceding claims, wherein the coating is a multifunctional coating.

9. Process according to claim 8, wherein the antimicrobial compound is combined with one or more agents that provides at least one functionality other than antimicrobial to the coating.

10. Process according to claim 8 or 9, wherein the multifunctional coating has at least one functionality selected from the group consisting of hydrophilic, hydrophobic, oleophobic, flame retardant or flame resistant, UV protection, bioactive and anti static.

11. Use of a plasma treatment in the preparation of an antimicrobial coating having the ability to destroy at least some types of micro-organisms for enhancing the antimicrobial properties of said coating, wherein said plasma treatment comprises a plasma assisted grafting technique, and wherein said plasma treatment is carried out under atmospheric pressure.

12. Use of a reactive antimicrobial active compound with general formula (I) or (II) 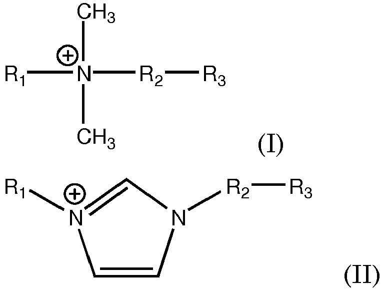 in a process according to any one of claims 1-10, wherein
R₁ is a C₃₋₁₂ alkyl group, preferably a C₅₋₉ alkyl group, more preferably a C₇ or C₈ alkyl group;
R₂ is an optionally substituted C₁₋₆ chain, preferably a C₂₋₄ chain; and
R₃ is chosen from the group consisting of an acrylate, a diisocyanate, a diol, an ethylene, and a styrene.

13. Use of a reactive antimicrobial active compound in a process according to any one of claims 1-10, wherein the antimicrobial active compound is selected from the antimicrobial active compounds defined in claim 6.

14. Substrate with an antimicrobial coating obtainable by a process according to any one of claims 1-10, wherein the antimicrobial active compound is selected from the antimicrobial active compounds defined in claim 6, preferably said substrate is chosen from the group consisting of textile, nanofibre non woven, felt, yarn, fibre, paper, foil, membranes, leather, polymers, wood and ceramics.

15. Substrate according to claim 14 in the form of a filter, anti allergic product, hygiene article or textile for medical purposes, such as bandages, medical garment, bed linen and surgical drape; sports textile, apparel textile, footwear, leather and technical textile, such as textile for interior design of automobile, ship, aviation and space travel; a carpet, textiles for business and home use, such as seat covers, curtains towels and linen; packaging material, and historical patrimony, such as for example historical textile, leather, parchment and manuscripts.

16. Use of a substrate according to claim 14 or 15 in building and/or construction, or as filling material, or for protection against biological and/or chemical hazards and/or for personal protection products, for example for police and military personnel.

## Patentansprüche

1. Verfahren zur Herstellung eines Substrats mit einer antimikrobiellen Beschichtung, umfassend:
- Bereitstellen eines Substrats;
- Aussetzen einer Oberfläche des Substrats einer Plasmaumgebung, wobei die Plasmaumgebung angelegt wird durch dielektrische Sperrschicht-Oberflächenentladungen; und
- Aufbringen einer antimikrobiellen, aktiven Verbindung mit der Fähigkeit, mindestens einige Arten von Mikroorganismen auf der Oberfläche des Substrats zu zerstören.

2. Verfahren nach Anspruch 1, wobei mindestens ein Teil der antimikrobiellen, aktiven Verbindung auf der Oberfläche des Substrats aufgebracht wird:
- gleichzeitig mit Aussetzen der Oberfläche des Substrats der Plasmaumgebung;
- nachdem die Oberfläche des Substrats der Plasmaumgebung ausgesetzt wurde; oder
- bevor die Oberfläche des Substrats mit der aufgetragenen antimikrobiellen, aktiven Verbindung der Plasmaumgebung ausgesetzt wird.

3. Verfahren nach einem der Ansprüche 2, wobei die antimikrobielle, aktive Verbindung polymerisiert ist.

4. Verfahren nach einem der vorhergehenden Ansprüche, wobei die antimikrobielle, aktive Verbindung einen quaternären Ammoniumrest umfasst.

5. Verfahren nach einem der vorhergehenden Ansprüche, wobei die antimikrobielle, aktive Verbindung einen optional substituierten aromatischen Heterozyklus mit mindestens einem Stickstoffatom umfasst, bevorzugt ausgewählt aus der Gruppe bestehend aus Imidazolen, Triazolen, Tetrazolen, Pyridinen, Pyrrolen, Pyrimidinen, Pyridazin, Pyrazin, Purin oder einem Derivat davon.

6. Verfahren nach einem der vorhergehenden Ansprüche, wobei die antimikrobielle, aktive Verbindung ausgewählt ist aus: 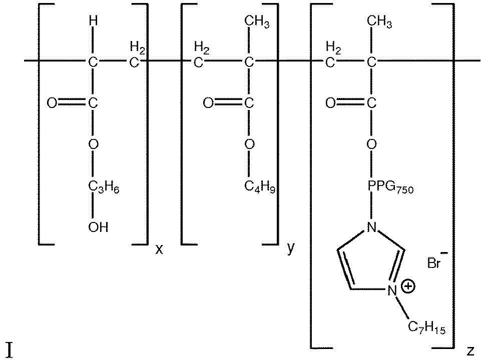 x = 2, y = 1 und z = 1; 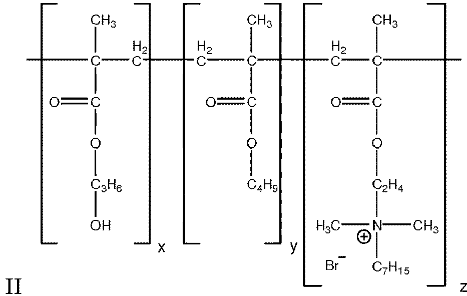 x = 1, y = 2 und z = 1; 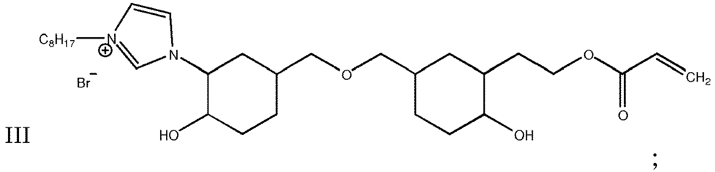 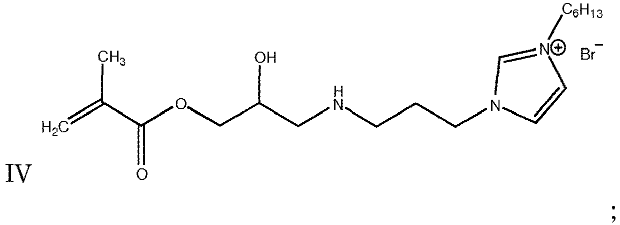 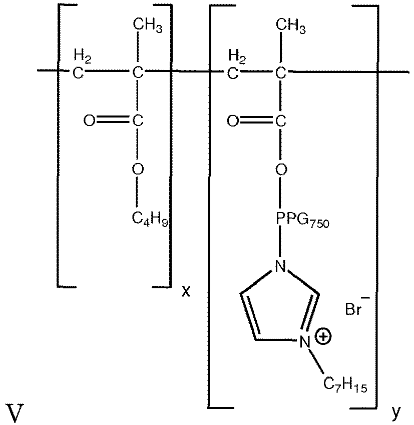 x = 2 und y = 1, PPG₇₅₀ = Polypropylenglykol mit einem MW von 750; 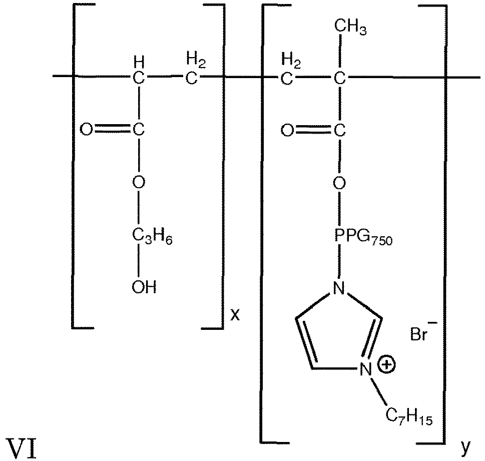 x = 1 oder 4 und y = 1, PPG₇₅₀ = Polypropylenglykol mit einem MW von 750; 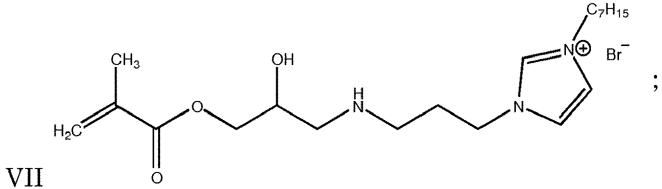 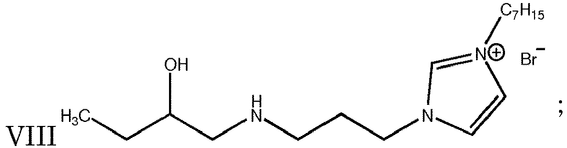 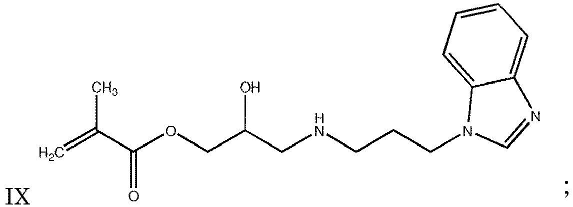 und 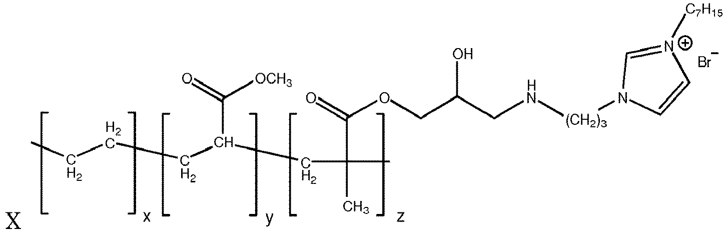 x = 5-15 %, y = 70-90 %, z = 5-15 %.

7. Verfahren nach einem der vorhergehenden Ansprüche, wobei die antimikrobielle, aktive Verbindung eine reaktive Gruppe umfasst, ausgewählt aus der Gruppe bestehend aus einem (Meth)acrylat oder einem Derivat davon, einem Hydroxyl oder einer Amingruppe, wobei bevorzugt die reaktive Gruppe durch einen Isocyanat-, Glycidyl-, einen Alkohol-, einen Epoxid-, einen Vinyl- oder einen Allylrest gebunden ist.

8. Verfahren nach einem der vorhergehenden Ansprüche, wobei die Beschichtung eine multifunktionelle Beschichtung ist.

9. Verfahren nach Anspruch 8, wobei die antimikrobielle Verbindung kombiniert ist mit einem Agens oder mehreren Agenzien, das/die der Beschichtung mindestens eine Funktionalität außer der mikrobiellen bietet/bieten.

10. Verfahren nach Anspruch 8 oder 9, wobei die multifunktionelle Beschichtung mindestens eine Funktionalität hat, ausgewählt aus der Gruppe bestehend aus hydrophil, hydrophob, oleophob, flammhemmend oder flammbeständig, UV-Schutz, bioaktiv und antistatisch.

11. Verwendung einer Plasmabehandlung bei der Herstellung einer antimikrobiellen Beschichtung mit der Fähigkeit, mindestens einige Arten von Mikroorganismen zu zerstören zur Verbesserung der antimikrobiellen Eigenschaften der Beschichtung, wobei die Plasmabehandlung ein plasmaunterstütztes Pfropfverfahren umfasst, und wobei die Plasmabehandlung unter Atmosphärendruck ausgeführt wird.

12. Verwendung einer reaktiven, antimikrobiellen, aktiven Verbindung der allgemeinen Formel (I) oder (II) 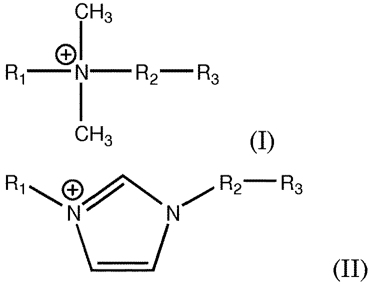 in einem Verfahren nach einem der Ansprüche 1-10, wobei
R₁ eine C₃₋₁₂ Alkylgruppe ist, bevorzugt eine C₅₋₉ Alkylgruppe, besonders bevorzugt eine C₇ oder C₈ Alkylgruppe;
R₂ eine optional substituierte C₁₋₆ Kette ist, bevorzugt eine C₂₋₄ Kette; und
R₃ ausgewählt ist aus der Gruppe bestehend aus einem Acrylat, einem Diisocyanat, einem Diol, einem Ethylen und einem Styrol.

13. Verwendung einer reaktiven, antimikrobiellen, aktiven Verbindung in einem Verfahren nach einem der Ansprüche 1-10, wobei die antimikrobielle, aktive Verbindung ausgewählt ist aus den antimikrobiellen, aktiven Verbindungen definiert in Anspruch 6.

14. Substrat mit einer antimikrobiellen Beschichtung, erhältlich durch ein Verfahren nach einem der Ansprüche 1-10, wobei die antimikrobielle, aktive Verbindung ausgewählt ist aus den antimikrobiellen, aktiven Verbindungen definiert in Anspruch 6, bevorzugt wobei das Substrat ausgewählt ist aus der Gruppe bestehend aus Textil, nichtgewebter Nanofaser, Filz, Garn, Faser, Papier, Folie, Membranen, Leder, Polymeren, Holz und Keramiken.

15. Substrat nach Anspruch 14 in Form eines Filters, antiallergischen Produkts, Hygieneartikels oder Textils für medizinische Zwecke, wie etwa Bandagen, medizinisches Kleidungsstück, Bettwäsche und chirurgisches Tuch; Sporttextil, Kleidungstextil, Schuhwerk, Leder und technisches Textil, wie etwa Textil für Innenausstattung von Automobil, Schiff, Luftfahrt und Raumfahrt; ein Teppich, Textilien zur geschäftlichen und privaten Verwendung, wie etwa Sitzbezüge, Vorhänge, Handtücher und Bettwäsche; Verpackungsmaterial und historisches Erbe, wie beispielsweise historisches Textil, Leder, Pergament und Handschriften.

16. Verwendung eines Substrats nach Anspruch 14 oder 15 beim Aufbau und/oder Konstruktion oder als Füllmaterial oder zum Schutz gegen biologische und/oder chemische Gefahren und/oder für persönliche Schutzprodukte, zum Beispiel für Polizei- und Militärpersonal.

## Revendications

1. Méthode de préparation d'un substrat ayant un revêtement antimicrobien consistant à :
- fournir un substrat ;
- soumettre une surface du substrat à un environnement de plasma, dans lequel ledit environnement de plasma est appliqué par des décharges de barrière diélectrique superficielle ; et
- déposer un composé antimicrobien actif ayant la capacité de détruire au moins certains types de micro-organismes sur la surface du substrat.

2. Méthode selon la revendication 1, dans lequel au moins une partie du composé antimicrobien actif est déposée sur la surface du substrat :
- simultanément à la soumission de la surface du substrat à l'environnement de plasma ;
- après avoir soumis la surface du substrat à l'environnement de plasma ; ou
- avant que la surface du substrat ayant le composé antimicrobien actif appliqué ne soit soumise à l'environnement de plasma.

3. Méthode selon l'une quelconque des revendications 2, dans laquelle le composé antimicrobien actif est polymérisé.

4. Méthode selon l'une quelconque des revendications précédentes, dans laquelle le composé antimicrobien actif comprend une fraction d'ammonium quaternaire.

5. Méthode selon l'une quelconque des revendications précédentes, dans laquelle le composé antimicrobien actif comprend un hétérocycle aromatique facultativement substitué avec au moins un atome d'azote, de préférence sélectionné parmi le groupe constitué de imidazoles, triazoles, tétrazoles, pyridines, pyrroles, pyrimidines, pyridazine, pyrazine, purine ou un dérivé de ceux-ci

6. Méthode selon l'une quelconque des revendications précédentes, dans laquelle le composé antimicrobien actif est sélectionné parmi : 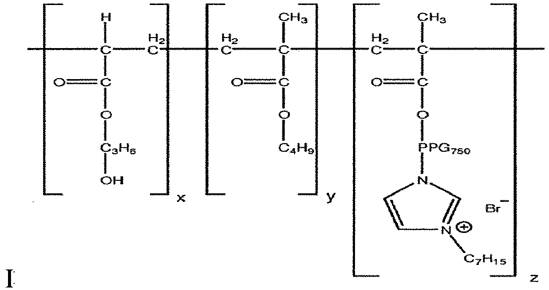 x=2, y=1 et z=1 ; 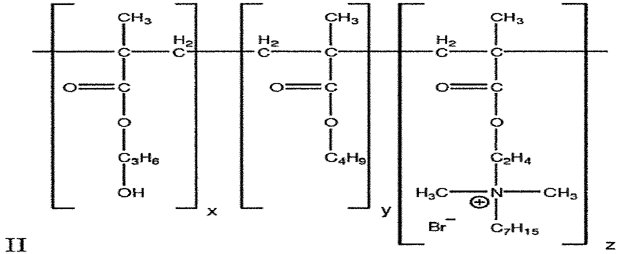 x=1, y=2 et z=1 ; 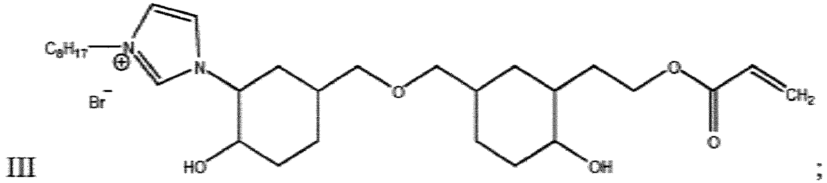 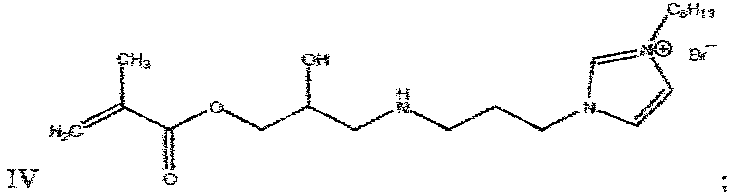 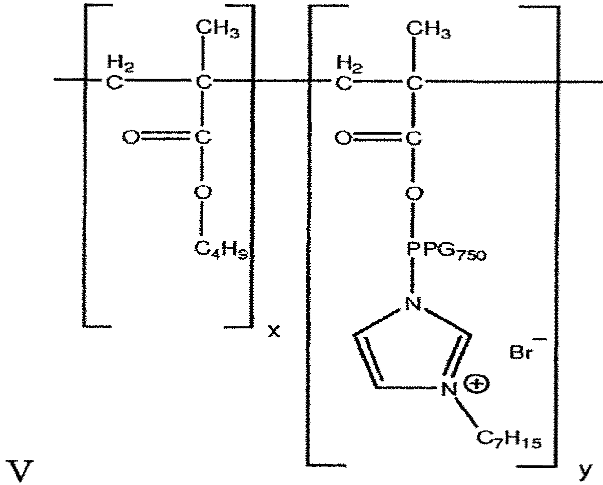 x=2 et y=1, PPG₇₅₀ = polypropilène glycol ayant un poids moléculaire de 750 ; 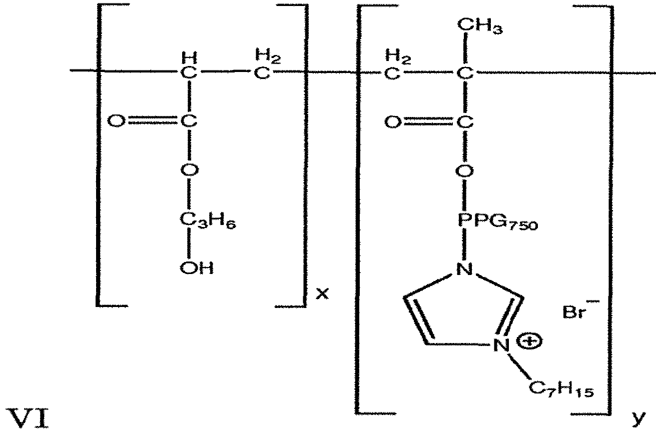 x =1 ou 4, et y =1, PPG₇₅₀ = polypropilène glycol ayant un poids moléculaire de 750 ; 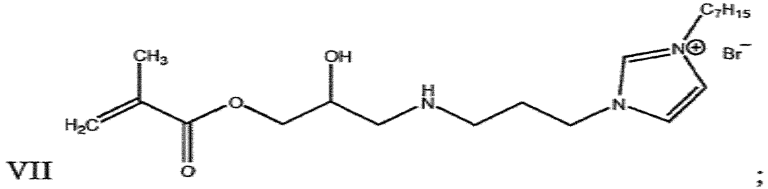 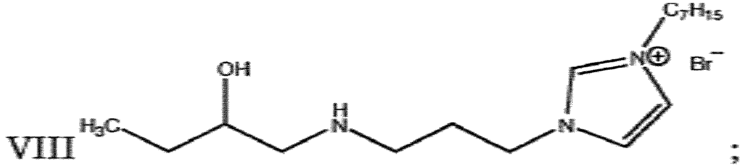 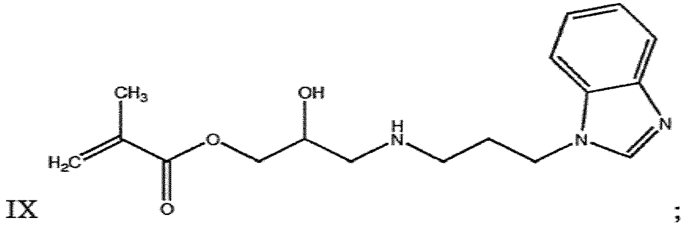 et 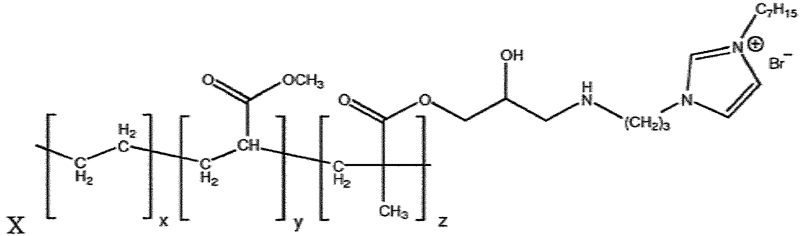 x= 5 à 15 %, y= 70 à 90 %, z=5 à 15 %

7. Méthode selon l'une quelconque des revendications précédentes, dans laquelle le composé antimicrobien actif comprend un groupe réactif sélectionné parmi le groupe constitué d'un (méth)acrylate ou d'un dérivé de celui-ci, d'un hydroxyle ou d'un groupe amino, de préférence le groupe réactif est lié par l'intermédiaire d'un isocyanate, de glycidyle, d'un alcool, d'un époxyde, d'un vinyle ou d'une fraction allyle.

8. Méthode selon l'une quelconque des revendications précédentes, dans laquelle le revêtement est un revêtement multifonctionnel.

9. Méthode selon la revendication 8, dans laquelle le composé antimicrobien est combiné avec un ou plusieurs agents qui fournissent au revêtement au moins une fonctionnalité autre qu'antimicrobien.

10. Méthode selon les revendications 8 ou 9, dans laquelle le revêtement multifonctionnel a au moins une fonctionnalité choisie parmi le groupe constitué d'hydrophilie, d'hydrophobie, d'oléophobie, de retardateur de flamme ou résistant à une flamme, de protection vis-à-vis des UV, bioactif et anti-statique.

11. Utilisation d'un traitement au plasma dans la préparation d'un revêtement antimicrobien ayant la capacité de détruire au moins certains types de microorganismes pour renforcer les propriétés antimicrobiennes dudit revêtement, dans laquelle ledit traitement au plasma comprend une technique de greffe assistée par plasma, et dans laquelle ledit traitement au plasma est effectué sous pression atmosphérique.

12. Utilisation d'un composé antimicrobien actif réactif ayant la formule générale (I) ou (II) 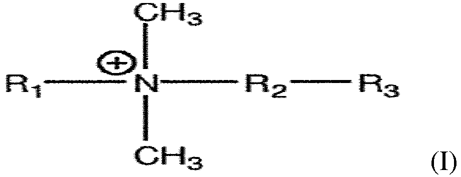 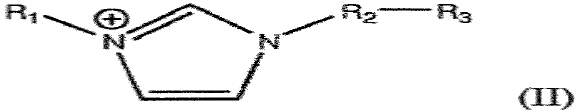
dans une méthode selon l'une quelconque des revendications 1 à 10, dans laquelle R₁ est un groupe alkyle C₃₋₁₂, de préférence un groupe alkyle C₅₋₉, de manière plus préférée un groupe alkyle C₇ ou C₈;
R₂ est une chaîne C₁₋₆ facultativement substituée, de préférence une chaîne C₂₋₄ ; et
R₃ est choisi parmi le groupe constitué d'un acrylate, d'un diisocyanate, d'un diol, d'un éthylène, et d'un styrène.

13. Utilisation d'un composé antimicrobien actif de réaction dans une méthode selon l'une quelconque des revendications 1 à 10, dans laquelle le composé antimicrobien actif est sélectionné parmi les composés antimicrobiens actifs définis dans la revendication 6.

14. Substrat ayant un revêtement antimicrobien pouvant être obtenu par une méthode selon l'une quelconque des revendications 1 à 10, dans lequel le composé antimicrobien actif est sélectionné parmi les composés antimicrobiens actifs définis dans la revendication 6, de préférence ledit substrat est choisi parmi le groupe constitué de textile, d'un non-tissé de nano fibres, de feutre, d'un fil, d'une fibre, de papier, d'une feuille, de membranes, de cuir, de polymères, de bois et de céramiques.

15. Substrat selon la revendication 14 ayant la forme d'un filtre, d'un produit anti allergique, d'un article d'hygiène ou d'un textile à des fins médicales, tels que des pansements, des vêtements médicaux, de linge de lit et drap chirurgicaux ; du textile pour sport, du textile d'habillement, des chaussures, du cuir et du textile technique, tel qu'un textile pour la conception de l'intérieur d'une automobile, d'un navire, d'aviation et de voyage dans l'espace ; un tapis, des textiles pour des bureaux et des utilisations domestiques, tels que des recouvrements de siège, des rideaux, des serviettes et du linge ; du matériau d'emballage, et un patrimoine historique, tel que par exemple un textile historique, du cuir, du parchemin et des manuscrits.

16. Utilisation d'un substrat selon les revendications 14 ou 15 dans le bâtiment et/ou la construction, ou comme matériau de remplissage, ou pour une protection vis-à-vis des risques biologiques et/ou chimiques et/ou des produits pour la protection personnelle, par exemple, du personnel de police et militaire.
